# EUROPÄISCHE PATENTSCHRIFT

(11) **EP 3 595 548 B1**
(45) Veröffentlichungstag und Bekanntmachung des Hinweises auf die Patenterteilung: **13.08.2025**
(21) Anmeldenummer: 18711086.1
(22) Anmeldetag: 12.03.2018
(51) Int. Cl.: A61B 17/28, B33Y 80/00

(54) **ADDITIV HERGESTELLTES MEDIZINTECHNISCHES INSTRUMENT SOWIE VERFAHREN ZUR HERSTELLUNG EINES SOLCHEN INSTRUMENTS**
ADDITIVELY MANUFACTURED MEDICAL INSTRUMENT AND METHOD FOR MANUFACTURING SUCH AN INSTRUMENT
INSTRUMENT MÉDICAL FABRIQUÉ PAR ADDITION ET PROCÉDÉ DE FABRICATION D'UN TEL INSTRUMENT

(30) Priorität: 16.03.2017 DE 102017105706
(43) Veröffentlichungstag der Anmeldung: 22.01.2020
(73) Patentinhaber: Aesculap AG, 78532 Tuttlingen (DE)
(72) Erfinder: WEISSHAUPT, Dieter, 78194 Immendingen (DE); KOLLER, Tobias, 78532 Tuttlingen (DE)
(74) Vertreter: Winter, Brandl - Partnerschaft mbB
(86) Internationale Anmeldenummer: PCT/EP2018/056116
(87) Internationale Veröffentlichungsnummer: WO 2018/166989

(56) Entgegenhaltungen:
- EP-A1- 2 873 381
- WO-A2-2012/021779
- US-A- 2 305 156
- US-A- 5 514 147
- US-A1- 2017 020 571
- WONG JULIELYNN Y. ET AL: "3D Printing of Surgical Instruments for Long-Duration Space Missions", AVIATION, SPACE AND ENVIRONMENTAL MEDICINE., vol. 85, no. 7, 1 July 2014 (2014-07-01), US, pages 758 - 763, XP093013927, ISSN: 0095-6562, DOI: 10.3357/ASEM.3898.2014
- WONG JULIELYNN Y. ET AL: "3D Printed Surgical Instruments Evaluated by a Simulated Crew of a Mars Mission", AEROSPACE MEDICINE AND HUMAN PERFORMANCE, vol. 87, no. 9, 1 September 2016 (2016-09-01), US, pages 806 - 810, XP093013928, ISSN: 2375-6314, DOI: 10.3357/AMHP.4281.2016
- CUMMINS ET AL: "The rise of additive manufacturing", INTERNET CITATION, 24 May 2010 (2010-05-24), XP002779796, Retrieved from the Internet <URL:https://www.theengineer.co.uk/issues/24-may-2010/the-rise-of-additive-manufacturing/> [retrieved on 20180406]

## Beschreibung

Die Erfindung betrifft ein medizintechnisches Instrument umfassend zumindest eine erste Instrumentenbranche und eine zweite Instrumentenbranche, die zueinander relativbewegbar sind, wobei die erste Instrumentenbranche als männliche Instrumentenbranche mit einem männlichen Lagerabschnitt ausgebildet ist, und die zweite Instrumentenbranche als weibliche Instrumentenbranche mit einem eine Durchgangsöffnung aufweisenden weiblichen Lagerabschnitt ausgebildet ist, wobei die erste Instrumentenbranche an der zweiten Instrumentenbranche angeordnet ist, indem der männliche Lagerabschnitt die Durchgangsöffnung des weiblichen Lagerabschnitts durchgreift. Sie betrifft des Weiteren ein Verfahren zum Herstellen eines medizintechnischen Instruments mittels eines additiven Fertigungsverfahrens.

Die Herstellung eines üblichen chirurgischen Instrumentes mit Durchsteckschluss ist in nachteiliger Weise verhältnismäßig aufwändig. Derartige Instrumente mit Durchsteckschluss sind beispielsweise Scheren, Klemmen, Zangen oder ähnliche Instrumente, die zwei Instrumentenbranchen/Instrumententeile aufweisen, die zueinander relativpositionierbar sind, insbesondere durch Verschwenken. Die beiden Instrumentenbranchen sind derart ausgebildet und montiert, dass eine von ihnen die andere durchdringt, man kann auch sagen dass die eine Branche durch die andere hindurchgesteckt ist, daher die Bezeichnung "Durchsteckschluss". Üblicherweise werden die beiden Instrumentenbranchen separat vorgefertigt, zum Beispiel durch Umformen und/oder spanende Bearbeitung. Die derart vorgefertigten Branchen werden anschließend durch Aufweiten eines Schlussbereiches der weiblichen Instrumentenbranche ineinander gesteckt und verpresst. Es können weitere Arbeitsgänge erforderlich sein, wie Bohren, Schleifen, Richten, Härten und/der Bürsten.

Bei der Herstellung solcher bekannter Instrumente ist von Nachteil, dass in der Regel ein hoher Aufwand mit zahlreichen Arbeitsgängen erforderlich ist. Dies führt dazu, dass die Fertigung kostenintensiv und werkzeugintensiv ist, kaum Automatisierungsmöglichkeiten bestehen und sogar ein hoher manueller Aufwand erforderlich sein kann. Ein weiterer Nachteil bekannter medizintechnischer Instrumente mit Durchsteckschluss ist, dass deren zueinander relativbewegbare Branchen in der Regel über eine (Schwenk-) Achse aneinander gekoppelt sind. Es gibt daher stets einen Bereich zwischen den Branchen, der zu Reinigungs- oder Desinfektionszwecken nur schlecht zugänglich ist, da, abgesehen von der angesprochenen Rotation zueinander, andere Möglichkeiten zur Relativpositionierung nicht bestehen. Beispiele für solche Instrumente sind beispielsweise in US 2017 / 0 020 571 A1, WO 2012 / 021 779 A2, EP 2 873 381 A1, US 5 514 147 A oder US 2 305 156 A1 offenbart.

Die der Erfindung vor diesem Stand der Technik zugrundeliegende Aufgabe ist, ein medizintechnisches Instrument bereit zu stellen, das in fertigungstechnischer Hinsicht nicht mit den vorstehend beschriebenen Problemen des Stands der Technik belastet ist. Dessen Fertigung soll insbesondere günstig sein, einen hohen Automatisierungsgrad aufweisen und vorzugsweise eine (weitgehend) werkzeugfreie Montage ermöglichen. Des Weiteren soll das Instrument im Hinblick auf die Reinigungs- und Sterilisierbarkeitseignung hin gegenüber dem Stand der Technik verbessert sein. Diese Aufgabe wird nach der Erfindung gelöst durch ein medizintechnisches Instrument mit den Merkmalen des Patentanspruchs 1 sowie ein Verfahren mit den Merkmalen des Patentanspruchs 8.

Insbesondere wird die Aufgabe also gelöst durch ein medizintechnisches Instrument nach Anspruch 1 aufweisend zumindest eine erste Instrumentenbranche und eine zweite Instrumentenbranche, die zueinander relativbewegbar sind.

Die erste Instrumentenbranche ist als männliche Instrumentenbranche mit einem männlichen Lagerabschnitt ausgebildet. Die zweite Instrumentenbranche ist als weibliche Instrumentenbranche mit einem eine Durchgangsöffnung aufweisenden weiblichen Lagerabschnitt (Durchsteck-Kasten) ausgebildet. Die erste Instrumentenbranche ist an der zweiten Instrumentenbranche angeordnet, indem der männliche Lagerabschnitt die Durchgangsöffnung (Durchsteck-Kasten) des weiblichen Lagerabschnitts durchgreift. Das erfindungsgemäße Instrument ist dadurch gekennzeichnet, dass es (zumindest die erste Instrumentenbranche) mittels eines additiven Fertigungsverfahrens hergestellt ist.

Im Hinblick auf ein Verfahren wird die Aufgabe gelöst durch das Verfahren nach Anspruch 8 zum Herstellen eines medizintechnischen Instruments, insbesondere eines erfindungsgemäßen Instruments, insbesondere eines Instruments nach einem der angehängten Ansprüche, mittels eines additiven Fertigungsverfahrens, wonach zumindest eine erste, männliche Instrumentenbranche mit einem männlichen Lagerabschnitt und eine zweite Instrumentenbranche als weibliche Instrumentenbranche mit einem eine Durchgangsöffnung aufweisenden weiblichen Lagerabschnitt (Durchsteck-Kasten) ausgebildet wird, wobei zumindest der männliche Lagerabschnitt die Durchgangsöffnung des weiblichen Lagerabschnitts durchgreifend additiv (entsprechend dem allgemein bekannten generativen/additiven Herstellungsverfahren) hergestellt wird.

Die Herstellung von zwei ineinander gesteckten Instrumentenbranchen mittels eines additiven Fertigungsverfahrens gelingt dadurch, dass beispielsweise in einer bestimmten geöffneten Stellung des Instruments der Abstand der beiden Instrumentenbranchen derart ist, dass die Branchen bei der Herstellung nicht miteinander verschmelzen und anschließend relativ zueinander bewegt/verschwenkt werden können. Erfindungsgemäß werden die Branchen derart hergestellt, dass sie separiert voneinander in einem einzigen Fertigungsschritt erzeugt werden. Das heißt, dass beide Branchen gleichzeitig hergestellt werden, wobei sie dabei sogleich in ihrer bestimmungsgemäßen Anordnung zueinander und Konstellation, also mit einem Durchsteckschluss, additiv oder generativ aufgebaut werden. Man kann auch sagen, dass anders als im Stand der Technik, wo die beiden Branchen zunächst separat voneinander erzeugt und nachfolgend miteinander montiert werden, nach der Erfindung die beiden Branchen während ihrer Erzeugung miteinander in der gewünschten Weise gekoppelt werden, insbesondere bestimmungsgemäß zueinander relativpositionierbar gekoppelt/ ineinander gesteckt sind. In vorteilhafter Weise entfällt damit der bislang erforderliche werkzeugintensive Endmontageschritt (das Ineinanderstecken) der beiden Branchen. Ein weiterer Vorteil ist, dass mit der Erfindung gegenüber aktuellen Herstellungsmethoden die Anzahl erforderlicher Fertigungsschritte deutlich reduziert werden kann, was Zeit und Kosten einspart. Das Instrument kann allerdings weiterhin nach der additiven Fertigung je nach den jeweils vorliegenden Anforderungen gehärtet, gebohrt, vernietet und/oder oberflächenbehandelt werden. Die Erfindung betrifft nahezu sämtliche Arten von chirurgischen Instrumenten mit Durchsteckschluss, also zum Beispiel Zangen, Klemmen, Scheren, etc.

Das Instrument nach der Erfindung besitzt bevorzugt einen Schlussbereich, in dem die beiden Branchen des Instruments miteinander verbunden oder gekoppelt sind. Dieser Schlussbereich / Koppelbereich / Verbindungsbereich ist gebildet durch den männlichen Lagerabschnitt (der außerdem als Koppelabschnitt, Verbindungsabschnitt oder Schlussabschnitt bezeichnet werden kann) der ersten Branche und den weiblichen Lagerabschnitt/Durchsteck-Kasten (der außerdem als Koppelabschnitt, Verbindungsabschnitt oder Schlussabschnitt bezeichnet werden kann) der zweiten Branche. Dabei wird der männliche Abschnitt in der Durchgangsöffnung des weiblichen Abschnitts aufgenommen bzw. durchdringt diesen.

Vorteilhafte Ausführungsformen der Erfindung sind auch in den Unteransprüchen beansprucht und werden nachfolgend näher erläutert.

Eine bevorzugte Ausführungsform der Erfindung sieht vor, dass der weibliche Lagerabschnitt die Durchgangsöffnung vollumfänglich umgibt oder umschließt. Dies ermöglicht eine besonders stabile und genaue Führung der beiden Branchen zueinander.

Die Erfindung sieht vor, dass zwischen dem männlichen Lagerabschnitt der ersten Instrumentenbranche und dem weiblichen Lagerabschnitt der zweiten Instrumentenbranche in einer relativen Stellung der ersten Instrumentenbranche zur zweiten Instrumentenbranche, die auch als Fertigungsstellung bezeichnet werden kann, da das Instrument in dieser Stellung gefertigt wird, Spiel vorliegt, derart, dass die erste Instrumentenbranche und die zweite Instrumentenbranche kontaktlos aneinander/ineinander anordbar sind. Die Lagerabschnitte beider Instrumententeile weisen insbesondere jeweils drei einzelne Teilbereiche auf: einen zentralen Lager- oder Schlussbereich, in dem sich die beiden Instrumentenbranchen in jeder Stellung (Funktionsstellung und Fertigungsstellung) zueinander überdecken, und beiderseits angrenzende laterale Lager- oder Schlussbereiche, in denen sich die beiden Branchen nur überdecken, wenn sie sich in einer anderen Winkelstellung (Funktionsstellung) zueinander befinden als in der Fertigungsposition, beispielsweise im geschlossenen Zustand des Instruments. Eine gegenseitige Kontaktierung der beiden Branchen im Rahmen der additiven Fertigung kann besonders einfach verhindert werden, indem nach einer bevorzugten Ausführungsform der in der Fertigungsstellung vom weiblichen Lagerabschnitt überdeckte Bereich des männlichen Lagerabschnitts eine geringere Höhe als die lichte Weite der Durchgangsöffnung aufweist. Die beiden Instrumententeile können insbesondere so konstruiert sein, dass sie in einer bestimmten Öffnungsstellung (Fertigungsstellung), beispielsweise zwischen 80° und 120°, im gesamten Schlussbereich einen Abstand voneinander haben und sich nicht gegenseitig kontaktieren oder berühren. Dies kann zum Beispiel erreicht werden, indem der zentrale Schlussbereich des männlichen Instrumententeils und/oder der zentrale Schlussbereich des weiblichen Instrumententeils dünner ausgebildet ist als die jeweiligen lateralen Schlussbereiche. Auf diese Weise berühren sich die zentralen Schlussbereiche der beiden Instrumententeile in keiner Stellung, so dass eine stoffliche Verbindung der beiden Instrumententeile bei der Fertigung vermieden wird. Während sich die lateralen Schlussbereiche überdecken und sogar berühren (unmittelbar oder mittelbar zum Beispiel mit nachfolgend näher beschriebenen Kontaktstrukturen oder Kontaktelementen), wenn das Instrument zu einem bestimmten Grad (Funktionsstellung) geschlossen ist, überdecken sie sich in der Fertigungsposition/-stellung nicht, sodass sie sich auch nicht berühren können.

Nach einer bevorzugten Ausführungsform liegt in der Fertigungsstellung zwischen der ersten Instrumentenbranche und der zweiten Instrumentenbranche ein Winkel von ca. 80° bis ca. 120°, vorzugsweise von ca. 85° bis ca. 115° und besonders bevorzugt von ca. 90° bis ca. 110° vor. Vorzugsweise befinden sich die Instrumentenbranchen in der Fertigungsstellung in einem solchen Winkel oder in einer solchen Stellung zueinander, die bei einem bestimmungsgemäßen Gebrauch des Instruments im Praxiseinsatz nicht oder nur mit geringer Wahrscheinlichkeit vorkommt. Auf diese Weise kann besonders einfach eine sichere Funktion des Instruments (gute Schwenkführung in der/den Funktionsstellung(en) sichergestellt werden.

Eine vorteilhafte Ausführungsform der Erfindung sieht vor, dass an der ersten Instrumentenbranche und/oder an der zweiten Instrumentenbranche Kontaktstrukturen ausgebildet sind. Diese dienen einer gegenseitigen Kontaktierung von erster Instrumentenbranche und zweiter Instrumentenbranche in einer von der Fertigungsstellung abweichenden Funktions-/Gebrauchsstellung. So kann trotz der fertigungstechnisch bedingten räumlichen Trennung der Branchen voneinander eine definierte, sichere und stabile Führung der Branchen zueinander und aneinander während des Praxiseinsatzes erzielt werden. Nach einer Ausführungsform besitzt die männliche Branche im Schlussbereich oder im männlichen Lagerabschnitt zumindest eine Kontaktstruktur in Form einer Erhebung oder eines Bänkchens, die beim Schließen des Instrumentes das Spiel im Instrumentenschluss reduziert. Vorzugsweise sind auf jeder Seite zwei solche Bänkchen oder Erhebungen ausgebildet oder angeordnet. Ein besonders effektiver Spielausgleich bei gleichzeitig leichter Betätigung kann nach der Erfindung erzielt werden, indem im Bereich der Erhebung oder des Bänkchens federnde Zungen ausgebildet sind. Es wird derart zwischen den Branchen eine Federkraft ausgeübt, über deren Höhe die Betätigungskraft und Führung des Instruments bestimmt ist. Bei einer im Nachfolgenden beschriebenen Ausführungsform mit einem Stopfen ist zweckmäßig, dass die Branchen mittels eines separaten Achselements, das entsprechende Aussparungen in den Branchen durchgreift, relativbewegbar zueinander aneinander angeordnet und befestigt sind, um eine eindeutige Bewegung der beiden Teile zueinander zu definieren.

Gemäß einer nicht mehr zur Erfindung gehörenden Ausgestaltung können die erste Instrumentenbranche und die zweite Instrumentenbranche mittels einer Durchsteckachse schwenkbar zueinander positioniert sein. In der Regel kann eine solche Durchsteckachse als Niet ausgebildet sein.

Erfindungsgemäß weist eine der Instrumentenbranchen einen sich in Richtung der anderen Instrumentenbranche erhebenden Führungsvorsprung auf, der zum schwenkbaren Positionieren der Instrumentenbranchen in eine entsprechend ausgebildete kreisbogenförmige Vertiefung an der anderen Instrumentenbranche eingreift (Kulissenführung). Insbesondere kann die Vertiefung am weiblichen Lagerabschnitt ausgebildet sein, insbesondere beidseitig. Der Führungsvorsprung kann am männlichen Lagerabschnitt ausgebildet sein, insbesondere beidseitig.

Es ist von besonderem Vorteil, dass bei einer solchen Ausführungsform auf eine Achse, Durchsteckachse oder einen Niet verzichtet werden kann. Dies führt zum einen dazu, dass das Instrument ein Bauteil weniger aufweist, so dass bei seiner Herstellung gegenüber einem Instrument mit Durchsteckachse mindestens ein Arbeitsschritt fortfällt. Ein weiterer wesentlicher und wichtiger Vorteil liegt darin, dass das Instrument mit definierter Schließkraft / Öffnungskraft / Betätigungskraft hergestellt werden kann. Dies liegt daran, dass das Zusammenwirken der männlichen und weiblichen Branche, insbesondere zwischen diesen infolge deren gegenseitiger Passung wirkende Kräfte definiert hergestellt werden können, was bei einem Instrument mit vernieteter Steckachse nur schwierig zu erreichen ist. Beim Nietvorgang werden nämlich in der Regel die beiden Seiten des Schlusskastens der männlichen Branche verformt, insbesondere zusammen gedrückt. Das Maß einer solchen Verformung ist in nachteiliger Weise nicht oder nur schwer definiert zu beeinflussen, so dass sich ein Nietprozess schlecht auf eine bestimmte Kraft (Schließkraft, Öffnungskraft, Betätigungskraft) validieren lässt. Diese Problematik geht in der Praxis soweit, dass bei einer Herstellung mittels mehrerer Nietmaschinen jede Maschine Instrumente mit anderem Gang erzeugt, obwohl sowohl die Maschinen als auch deren Parameter identisch sind. Die Folge hiervon sind Instrumente, die sich voneinander deutlich im Gang (Betätigungskraft) unterscheiden, was nur durch umfangreiche Nacharbeiten soweit korrigierbar ist, dass eine annähernd gleiche Schließkraft, d.h. einen annähernd gleichen Gang erreicht werden kann. Dass die vorstehende Problematik bei einer Ausführungsform mit Kulissenführung vermieden werden kann, ist ein großer Vorteil. Die Vorsprünge und Nuten, insbesondere kreisbogenförmige Vorsprünge und korrespondierende kreisbogenförmige Nuten können insbesondere während der additiven Fertigung, insbesondere während eines Lasersintervorgangs, insbesondere an den gegenüberliegenden Flächen der lateralen Schlussbereiche oder Lagerabschnitte des männlichen und des weiblichen Instrumententeils ausgebildet werden. Diese sind konzentrisch zu dem beabsichtigten Drehzentrum der beiden Instrumentenbranchen angeordnet. Es ist von besonderem Vorteil, wenn die Nuten und Vorsprünge zudem mit Anlaufschrägen versehen sind, sodass der jeweilige Vorsprung trichterartig in die zugehörige Nut eingeführt werden kann, wenn die beiden Branchen von der Fertigungsposition in die Gebrauchsposition bewegt werden. Durch die Konzentrizität der Nuten und Vorsprünge erübrigt sich eine Achse als separates Bauteil und es können Fertigungsschritte eingespart werden. Die Vorsprünge können in Form von durchgehenden Kreisbögen oder in Form eines Vorsprungs oder mehrerer einzelner Vorsprünge ausgebildet sein, die auf einem entsprechenden Kreisbogen angeordnet sind. Eine Ausführungsform ist dadurch gekennzeichnet, dass Nuten und/oder Vorsprünge mit einer reibungsmindernden Schicht versehen sind. Alternativ können sie aus einem geeigneten Material, wie zum Beispiel PEEK, PE, etc., gefertigt sein und an den lateralen Schlussabschnitten montierbar sein. Außerdem können Schrägflächen an den lateralen Enden der kreisbogenförmigen Nut und/oder dem korrespondierenden Vorsprung vorgesehen sein. Diese bilden eine Art Einlauftrichter aus, der ein Einlaufen des Vorsprungs in die Nut erleichtert. Die Schrägflächen können außerdem derart ausgestaltet sein, dass sich der wirkliche Schluss (d.h. es gibt kein Wackeln zwischen den beiden Teilen mehr) erst kurz vor der Schließstellung des Instruments ausbildet.

Nach einer bevorzugten Ausführungsform weist das Instrument eine Hemmstruktur auf. Diese dient dazu, dass das Instrument beim Gebrauch aus einer seiner Funktions-/Gebrauchsstellungen nicht ohne weiteres oder versehentlich in die Fertigungsstellung gebracht werden kann. Die Hemmstruktur ist daher derart ausgebildet, dass bei einem Überführen des Instruments aus einer Gebrauchsstellung in die Fertigungsstellung eine Hemmung der Relativbewegung beider Instrumentenbranchen bewirkt wird (Bewegungswiderstand/Schwelle), die durch eine erhöhte nutzerseitige Betätigungskraft zu überwinden ist. Man kann auch sagen, dass auf diese Weise ein überwindbarer Anschlag für eine Öffnungsbewegung des Instruments ausgebildet wird. Dies hat den Vorteil, dass sich das Instrument bei einer regulären Benutzung nicht über einen bestimmten Punkt hinaus öffnet und somit verhindert wird, dass es versehentlich in die Fertigungsstellung überführt wird. Dennoch ist es möglich, das Instrument einfach in die Fertigungsstellung zu bringen, indem nämlich eine die Hemmung überwindende Betätigungskraft ausgeübt wird. In der Fertigungsstellung sind die Instrumentenbranchen (in Grenzen) in mehreren Richtungen zueinander verschiebbar und/oder verdrehbar, was eine einfache und effektive Reinigung, Instandhaltung und Desinfektion erleichtert. Zugleich wird sichergestellt, dass im Rahmen einer regulären Benutzung des Instruments die Fertigungsstellung nicht unbeabsichtigt erreicht wird. Die Hemmung oder der überwindbare Anschlag kann zum Beispiel durch Ausbilden unterschiedlicher Nuttiefen bewirkt werden. Alternativ kann sie bewirkt werden, indem Vertiefungen und Vorsprünge derart platziert sind, dass sich wahlweise miteinander in Eingriff gebracht werden können, d.h. in Abhängigkeit vom Öffnungswinkel der Branchen. Zum Beispiel können mehrere konzentrische korrespondierende Nuten und Vorsprünge vorgesehen sein. Für den Fall, dass die virtuelle Schwenkachse mittels Nuten und Vorsprüngen gebildet wird, können sich die restlichen Bereiche der lateralen Schlussflächen lediglich teilweise oder gar nicht berühren.

Die Branchen können insbesondere aus Metall und/oder Keramik bestehen/hergestellt sein.

Nach einer bevorzugten Ausführungsform kann in zumindest einer der Branchen, insbesondere in zumindest einem der Lagerabschnitte, eine Aussparung ausgebildet sein. In diese kann ein Stopfen eigepresst sein oder werden, der den Gang oder die Betätigungskraft des Instruments bestimmt. Der Stopfen kann aus einem anderen Material bestehen als die Instrumententeile, beispielsweise aus einem Kunststoff wie PEEK, PE, PA oder einem anderen metallischen Werkstoff, wie zum Beispiel austenitischem Stahl.

Der (seitliche) Abstand der beiden zentralen Schlussbereiche / Lagerabschnitte voneinander hängt von der Fertigungsgenauigkeit der zur additiven Fertigung verwendeten Maschinerie ab. Er liegt vorzugsweise in einem Bereich zwischen 0,05 mm und 0,3 mm. Die beiden Instrumententeile können so aufgebaut sein, dass sie bereits in einem 3D CAD Modell ineinander stecken. Dieses 3D CAD Modell kann Grundlage für eine zur additiven Fertigung genutzten Maschine sein, zum Beispiel eine Lasersintermaschine.

Man kann sagen, dass mit der Erfindung es erstmals möglich ist, chirurgische Instrumente mit einen additiven oder generativen Fertigungsverfahren herzustellen, ohne dass die Instrumententeile oder Instrumentenbranchen sich im Durchsteckschluss miteinander verbinden, insbesondere stoffschlüssig miteinander verbinden oder verschmelzen. Das gesamte Instrument wird mittels eines generativen bzw. additiven Fertigungsverfahrens hergestellt. Die Erfindung betrifft sowohl ein Fertigungsverfahren für derartige Instrumente sowie die Instrumente mit einem entsprechend dafür ausgebildeten Durchsteckschluss selbst.

Die Erfindung wird nachfolgend beispielhaft mit Hilfe von Zeichnungen näher erläutert. Es zeigt dabei:
- Fig. 1: eine weibliche Instrumentenbranche in zwei unterschiedlichen perspektivischen Ansichten,
- Fig. 2: eine männliche Instrumentenbranche in einer perspektivischen Ansicht,
- Fig. 3: ein vergrößertes Detail der männlichen Instrumentenbranche im Bereich des Lagerabschnitts,
- Fig. 4: ein Instrument in einer Aufsicht in der Fertigungsstellung,
- Fig. 5: vergrößert und in einem Schnitt den Bereich beider Lagerabschnitte in der Fertigungsstellung,
- Fig. 6: den Bereich beider Lagerabschnitte in der Fertigungsstellung in einer perspektivischen Ansicht,
- Fig. 7: den Bereich beider Lagerabschnitte einer Ausführungsform in der Fertigungsstellung in einer perspektivischen Ansicht,
- Fig. 8: den Bereich beider Lagerabschnitte einer Ausführungsform in der Fertigungsstellung in einer perspektivischen Ansicht und
- Fig. 9: den Bereich beider Lagerabschnitte einer Ausführungsform in der Fertigungsstellung in einer perspektivischen Ansicht.

Die Zeichnungen sind lediglich schematischer Natur und dienen nur dem Verständnis der Erfindung.

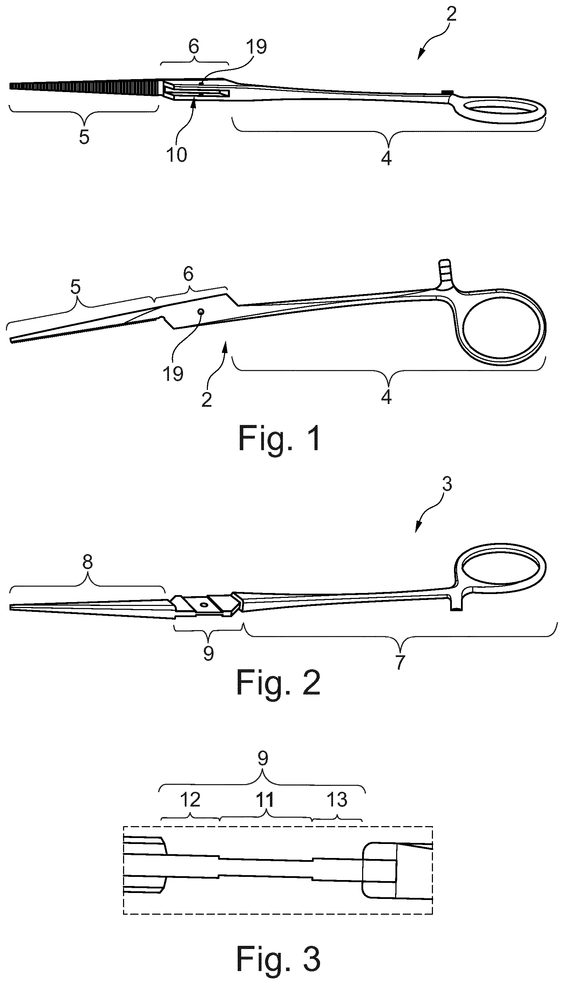

Die Figuren 1 und 2 zeigen als Beispiel für ein Instrument 1 nach der Erfindung eine chirurgische Klemme 1. Figur 1 zeigt dessen weibliche Branche 2, während Figur 2 dessen männliche Branche 3 zeigt. Die weibliche Branche 2 besitzt einen Griffabschnitt 4, einen Klemmenabschnitt 5 und einen zwischen diesen angeordneten weiblichen Lagerabschnitt (Durchsteck-Kasten) 6. In gleicher Weise besitzt die männliche Branche 3 einen Griffabschnitt 7, einen Klemmenabschnitt 8 und einen zwischen diesen angeordneten männlichen Lagerabschnitt 9. Der weibliche Lagerabschnitt 6 weist eine Durchsteck-/Durchgangsöffnung 10 auf. Es wird ausdrücklich darauf hingewiesen, dass die Darstellung der Figuren 1 und 2 nur zur besseren Darstellung und Erläuterung der beiden Branchen 2, 3 erfolgt, dass aber die erfindungsgemäße Klemme 1, die mittels eines erfindungsgemäßen additiven oder generativen Fertigungsverfahren hergestellt ist, nicht demontiert/montiert werden kann, also die beiden Branchen 2, 3 nicht ohne zerstört zu werden voneinander getrennt werden können.

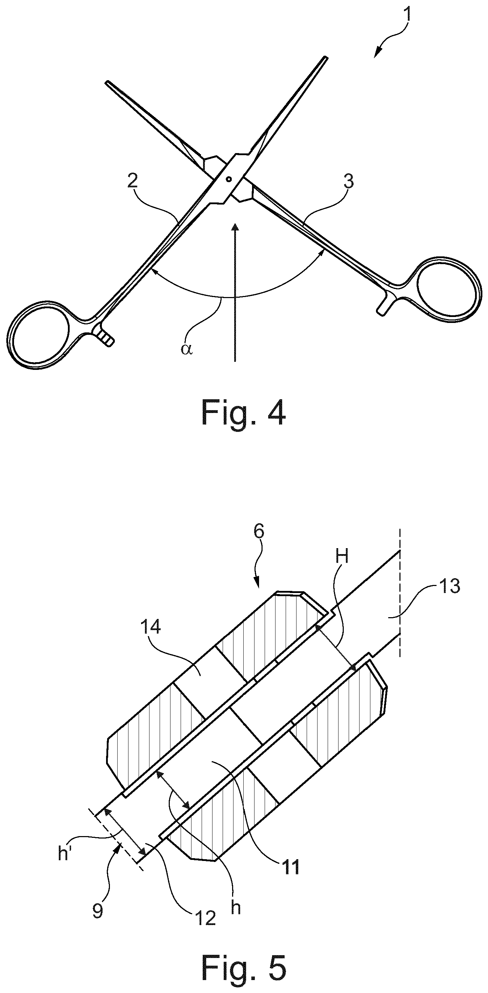

Figur 3 zeigt den männlichen Lagerabschnitt 9 vergrößert in einer seitlichen Ansicht. Es ist dargestellt, dass der männliche Lagerabschnitt 9 drei einzelne Teilbereiche aufweist: einen zentralen Lager- oder Schlussbereich 11, in dem sich die beiden Instrumentenbranchen 2, 3 in jeder Stellung zueinander überdecken, und beiderseits angrenzende laterale Lager- oder Schlussbereiche 12 bzw. 13, in denen sich die beiden Branchen 2, 3 nur überdecken, wenn sie sich in einer anderen Winkelstellung zueinander befinden als in der Fertigungsposition (gezeigt in Figur 4), beispielsweise im geschlossenen Zustand des Instruments 1. Der zentrale männliche Lagerabschnitt 11 weist einen Höhe h auf, die kleiner ist als die Höhe H der Durchgangsöffnung 10 des weiblichen Lagerabschnitts, siehe insbesondere in Figur 5. Infolge dieser Ausbildung besteht zwischen der männlichen Branche 3 und der weiblichen Branche 2 in der Fertigungsstellung ein (Doppel-) Spalt mit einer jeweiligen Spalthöhe von zum Beispiel 0,05mm bis 0,3mm und somit kein Kontakt. Die beiden Branchen 2, 3 lassen sich daher in der Fertigungsstellung durch ein additives oder generatives Verfahren wie zum Beispiel Lasersintern herstellen, ohne dass sie miteinander verbunden werden oder verbacken, so dass eine Relativbeweglichkeit der Branchen 2, 3 zueinander sichergestellt ist.

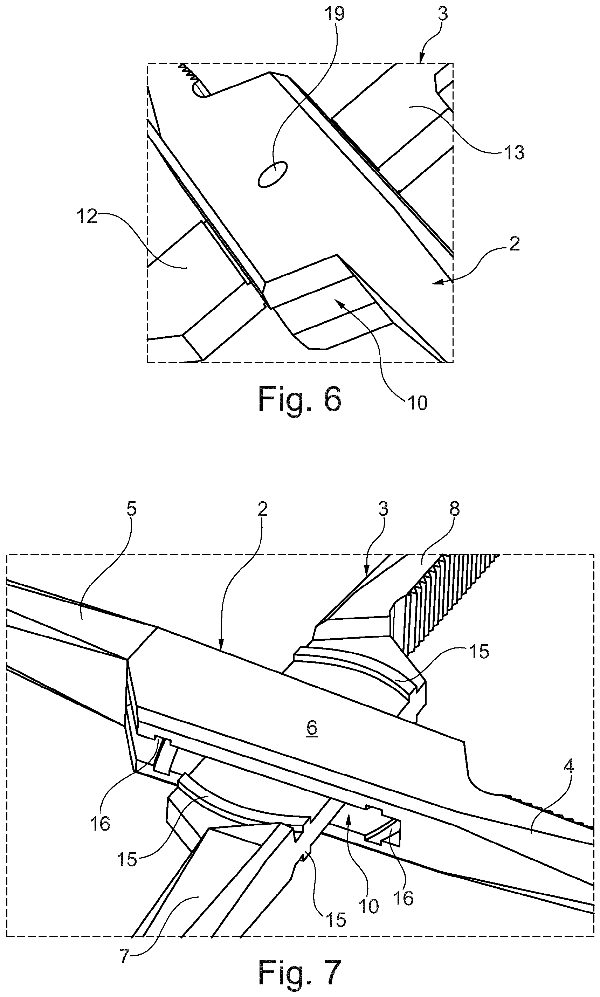

Um eine erwünschte gegenseitige Führung der Branchen 2, 3 beim Gebrauch des Instruments 1 zu erzielen, sind die lateralen Lager- oder Schlussbereiche 12, 13 nach der in Figur 6 gezeigten Ausführungsform mit gegenüber dem zentralen Lagerabschnitt 11 vergrößerten Höhe h' ausgebildet. Die Höhe h' entspricht im Wesentlichen der Höhe H der Durchgangsöffnung 9, so dass sich die beiden Branchen 2, 3 bei einer von der Fertigungsstellung abweichenden Stellung, also in einer Gebrauchsstellung, gegeneinander abstützen und derart die gewünschte Führung bewirkt wird.

Es sei darauf hingewiesen, dass die additive Fertigung des Instruments 1 in der in Figur 4 gezeigten Fertigungsstellung erfolgt, wobei die beiden Branchen 2, 3 im durchgesteckten Zustand additiv aufgebaut werden. Es werden also nicht die Branchen 2, 3 einzeln hergestellt und dann miteinander verbunden / durchgesteckt, sondern ihr additiver Aufbau erfolgt gleichzeitig zusammen mit dem gewünschten Durchsteckschluss. Figur 4 zeigt eine Fertigungsstellung, in der der Winkel α zwischen den Branchen 2, 3 etwa 90° beträgt.

Während die beiden Branchen 2, 3 der Ausführungsform der Figur 6 mit einer Durchsteckachse 14 miteinander schwenkbar verbunden sind, die durch entsprechende Achslöcher 19 in den Branchen 2, 3 gesteckt ist, zeigt Figur 7 eine davon abweichende Ausführungsform ohne eine solche Achse. Der männliche Lagerabschnitt 9 ist beidseitig mit jeweils zwei im Wesentlichen kreisbogenförmigen Erhebungen 15 versehen. Der weibliche Lagerabschnitt 6 ist entsprechend auf beiden Seiten der Durchgangsöffnung 10 mit kreisbogenförmigen Vertiefungen 16 versehen. Die Erhebungen 15 und die Vertiefungen 16 sind zum Ineinandergreifen ausgebildet und bestimmt, derart, dass sie eine Führungsstruktur oder Führungsbahn ausbilden, mittels der die beiden Branchen 2, 3 bei Verschwenkungen im Funktionsstellungsbereich zueinander geführt sind. Befinden sich die Branchen 2, 3 nicht in einer Funktionsstellung, sondern in der Fertigungsstellung, sind die Vertiefungen 16 und Erhebungen 15 außer Eingriff, so dass kein Kontakt zwischen den Branchen 2, 3 vorliegt.

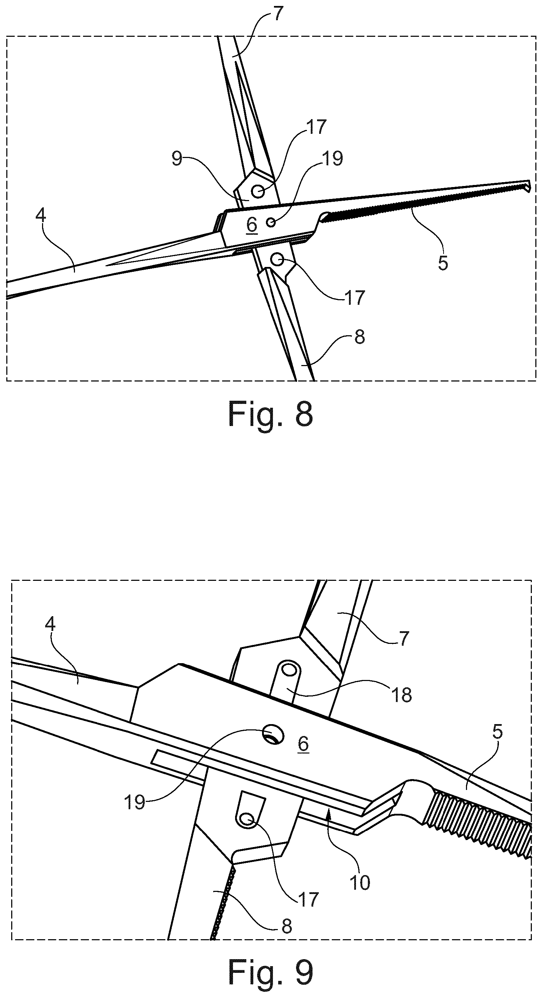

Figur 8 zeigt eine Ausführungsform des Instruments 1, bei der am männlichen Lagerschnitt 9 Kontaktstrukturen in Form von vorzugsweise in Vertiefungen eingebrachten oder eingepressten Stopfen 17 ausgebildet sind (können und in Form von Noppen aufgeklebt sein). Die Stopfen 17 sind dafür ausgebildet den Gang oder die Betätigungskraft des Instruments 1 zu bestimmen und können aus einem anderen Material bestehen als die Instrumententeile, beispielsweise aus einem Kunststoff wie PEEK, PE, PA oder einem anderen metallischen Werkstoff, wie zum Beispiel austenitischem Stahl.

Figur 9 zeigt eine weitere Ausführungsform, bei der die Kontaktstrukturen in Form von federnden Zungen 18 ausgebildet sind, an deren freien Enden bevorzugt Stopfen oder Noppen 17 gemäß der Fig. 8 angeordnet sein können.

Es sei zudem darauf hingewiesen, dass die Ausführungsform ohne Lagerachse nach Figur 7 mit den Kontaktstrukturen nach Figuren 8 oder 9 kombinierbar ist. Auch ist für die achslose Ausführungsform nach Figur 7 ein gemäß Figur 3 ausgebildeter Lagerbereich vorteilhaft verwendbar.

### Bezugszeichenliste

- 1: Instrument, Klemme
- 2: weibliche Branche / Instrumententeil
- 3: männliche Branche / Instrumententeil
- 4: Griffabschnitt
- 5: Klemmenabschnitt / Schneidabschnitt
- 6: weiblicher Lagerabschnitt / Durchsteckkasten
- 7: Griffabschnitt
- 8: Klemmenabschnitt / Schneidabschnitt
- 9: männlicher Lagerabschnitt / Durchsteckteil
- 10: Durchgangsöffnung / Längsschlitz
- 11: zentraler Lagerabschnitt (des Durchsteckteils)
- 12: lateraler Lagerabschnitt / Kontaktstruktur (des Durchsteckteils)
- 13: lateraler Lagerabschnitt / Kontaktstruktur (des Durchsteckteils)
- 14: Durchsteckachse / Niet
- 15: Erhebung
- 16: Vertiefung
- 17: Stopfen / Kontaktstruktur / Bolzen / Pin / Noppe
- 18: Federzunge / Kontaktstruktur
- 19: Achsloch
- α: Winkel
- h: Höhe
- h': Höhe
- H: Höhe

## Patentansprüche

1. Medizintechnisches Instrument (1) umfassend zumindest eine erste Instrumentenbranche (3) und eine zweite Instrumentenbranche (2), die zueinander relativbewegbar sind, wobei die erste Instrumentenbranche (3) als männliche Instrumentenbranche (3) mit einem männlichen Lagerabschnitt (9) ausgebildet ist, und die zweite Instrumentenbranche (2) als weibliche Instrumentenbranche (2) mit einem eine Durchgangsöffnung (10) aufweisenden weiblichen Lagerabschnitt (6) ausgebildet ist, wobei die erste Instrumentenbranche (3) an der zweiten Instrumentenbranche (2) angeordnet ist, indem der männliche Lagerabschnitt (9) die Durchgangsöffnung (10) des weiblichen Lagerabschnitts (6) durchgreift, wobei das Instrument (1) mittels eines additiven Fertigungsverfahrens hergestellt ist, und zwischen dem männlichen Lagerabschnitt (9) der ersten Instrumentenbranche (3) und dem weiblichen Lagerabschnitt (6) der zweiten Instrumentenbranche (2) in einer relativen Stellung der ersten Instrumentenbranche (3) zur zweiten Instrumentenbranche (2) Spiel vorliegt, derart, dass die erste Instrumentenbranche (3) und die zweite Instrumentenbranche (2) kontaktlos aneinander angeordnet sind, wobei eine der Instrumentenbranchen (2, 3) einen sich in Richtung der anderen Instrumentenbranche (2, 3) erhebenden Führungsvorsprung (15) aufweist, der zum schwenkbaren Positionieren der Instrumentenbranchen (2, 3) in eine entsprechend ausgebildete kreisbogenförmige Vertiefung (16) an der anderen Instrumentenbranche (2, 3) eingreift.

2. Medizintechnisches Instrument (1) nach Anspruch 1, **dadurch gekennzeichnet, dass** der weibliche Lagerabschnitt (6) die Durchgangsöffnung (10) vollumfänglich umgibt.

3. Medizintechnisches Instrument (1) nach einem der Ansprüche 1 oder 3, **dadurch gekennzeichnet, dass** der in der Fertigungsstellung vom weiblichen Lagerabschnitt (6) überdeckte Bereich (11) des männlichen Lagerabschnitts (9) eine geringere Höhe (h) als die Durchgangsöffnung (10) aufweist.

4. Medizintechnisches Instrument (1) nach einem der vorstehenden Ansprüche,
**dadurch gekennzeichnet, dass** in der Fertigungsstellung zwischen der ersten Instrumentenbranche (3) und der zweiten Instrumentenbranche (2) ein Winkel α von ca. 80° bis ca. 120°, vorzugsweise von ca. 85° bis ca. 115° und besonders bevorzugt von ca. 90° bis ca. 110° vorliegt.

5. Medizintechnisches Instrument (1) nach einem der vorstehenden Ansprüche,
**dadurch gekennzeichnet, dass** an der ersten Instrumentenbranche (3) und/oder an der zweiten Instrumentenbranche (2) Kontaktstrukturen (12, 13, 17, 18) ausgebildet sind, zur gegenseitigen Kontaktierung von erster Instrumentenbranche (3) und zweiter Instrumentenbranche (2) in einer von der Fertigungsstellung abweichenden Gebrauchsstellung.

6. Medizintechnisches Instrument (1) nach Anspruch 1, **dadurch gekennzeichnet, dass** die Vertiefung (16) am weiblichen Lagerabschnitt (6), insbesondere beidseitig, ausgebildet ist, und der Führungsvorsprung (15) am männlichen Lagerabschnitt (9), insbesondere beidseitig, ausgebildet ist.

7. Medizintechnisches Instrument (1) nach einem der vorstehenden Ansprüche,
**dadurch gekennzeichnet, dass** dieses eine Hemmstruktur aufweist, die derart ausgebildet ist, dass bei einem Überführen des Instruments (1) aus einer Gebrauchsstellung in die Fertigungsstellung eine Hemmung der Relativbewegung beider Instrumentenbranchen (2, 3) bewirkt wird, die durch eine erhöhte nutzerseitige Betätigungskraft zu überwinden ist.

8. Verfahren zum Herstellen eines medizintechnischen Instruments (1) mittels eines additiven Fertigungsverfahrens, wobei zumindest eine erste, männliche Instrumentenbranche (3) mit einem männlichen Lagerabschnitt (9) und eine zweite Instrumentenbranche (2) als weibliche Instrumentenbranche (2) mit einem eine Durchgangsöffnung (10) aufweisenden weiblichen Lagerabschnitt (6) ausgebildet wird, wobei der männliche Lagerabschnitt (9) die Durchgangsöffnung (10) des weiblichen Lagerabschnitts (6) durchgreifend additiv hergestellt wird, zwischen dem männlichen Lagerabschnitt (9) der ersten Instrumentenbranche (3) und dem weiblichen Lagerabschnitt (6) der zweiten Instrumentenbranche (2) in einer relativen Stellung der ersten Instrumentenbranche (3) zur zweiten Instrumentenbranche (2) Spiel vorliegt, derart, dass die erste Instrumentenbranche (3) und die zweite Instrumentenbranche (2) kontaktlos aneinander angeordnet sind, und wobei die Lagerabschnitte (6, 9) beider Instrumentenbranchen (2, 3) jeweils einen zentralen Lagerbereich (11), in dem sich die beiden Instrumentenbranchen (2, 3) in einer Funktionsstellung und einer Fertigungsstellung überlappen, und beiderseits angrenzende laterale Lagerbereiche (12, 13) aufweisen, in denen sich die beiden Instrumentenbranchen (2, 3) nur überlappen, wenn sie sich in einer anderen Winkelstellung als in der Fertigungsstellung befinden.

9. Verfahren nach Anspruch 8, **dadurch gekennzeichnet, dass** das Instrument (1) mittels Lasersintern hergestellt wird.

10. Verfahren nach Anspruch 8 oder 9, **dadurch gekennzeichnet, dass** zwischen der ersten Instrumentenbranche (3) und der zweiten Instrumentenbranche (3) ein Spalt von ca. 0,3mm bis 0,5mm erzeugt wird.

## Claims

1. Medical instrument (1) comprising at least a first instrument branch (3) and a second instrument branch (2) which are movable relative to each other, wherein the first instrument branch (3) is formed as a male instrument branch (3) with a male bearing portion (9), and the second instrument branch (2) is formed as a female instrument branch (2) with a female bearing portion (6) having a through opening (10), wherein the first instrument branch (3) is arranged on the second instrument branch (2) in that the male bearing portion (9) penetrates the through opening (10) of the female bearing portion (6), wherein the instrument (1) is manufactured by means of an additive manufacturing method, and there is play between the male bearing portion (9) of the first instrument branch (3) and the female bearing portion (6) of the second instrument branch (2) in a relative position of the first instrument branch (3) to the second instrument branch (2), in such a way that the first instrument branch (3) and the second instrument branch (2) are arranged without contact to each other, **wherein** one of the instrument branches (2, 3) has a guiding projection (15) rising in the direction of the other instrument branch (2, 3), which engages in a corresponding circular arc-shaped recess (16) on the other instrument branch (2, 3) for the pivotable positioning of the instrument branches (2, 3).

2. Medical instrument (1) according to claim 1, **characterized in that** the female bearing portion (6) completely surrounds the through opening (10).

3. Medical instrument (1) according to one of claims 1 or 3, **characterized in that** the area (11) of the male bearing portion (9) covered by the female bearing portion (6) in the manufacturing position has a height (h) which is lower than the through opening (10).

4. Medical instrument (1) according to one of the preceding claims,
**characterized in that** there is an angle α of approx. 80° to approx. 120°, preferably of approx. 85° to approx. 115°, and particularly preferably of approx. 90° to approx. 110° between the first instrument branch (3) and the second instrument branch (2) in the manufacturing position.

5. Medical instrument (1) according to one of the preceding claims,
**characterized in that** contact structures (12, 13, 17, 18) are formed on the first instrument branch (3) and/or on the second instrument branch (2) to establish mutual contact between the first instrument branch (3) and the second instrument branch (2) in a usage position that deviates from the manufacturing position.

6. Medical instrument (1) according to one of claim 1, **characterized in that** the recess (16) is formed on the female bearing portion (6), in particular on both sides, and the guiding projection (15) is formed on the male bearing portion (9), in particular on both sides.

7. Medical instrument (1) according to one of the preceding claims,
**characterized in that** it has an inhibition structure which is designed in such a way that, when the instrument (1) is transferred from a usage position to the manufacturing position, the relative movement of both instrument branches (2, 3) is inhibited, which must be overcome by an increased actuating force by the user.

8. Method of manufacturing a medical instrument (1) by means of an additive manufacturing method, wherein at least a first, male instrument branch (3) is formed with a male bearing portion (9) and a second instrument branch (2) is formed as a female instrument branch (2) with a female bearing portion (6) having a through opening (10), wherein the male bearing portion (9) is manufactured additively so as to penetrate the through opening (10) of the female bearing portion (6), there is play between the male bearing portion (9) of the first instrument branch (3) and the female bearing portion (6) of the second instrument branch (2) in a relative position of the first instrument branch (3) to the second instrument branch (2), in such a way that the first instrument branch (3) and the second instrument branch (2) are arranged without contact to each other, and wherein the bearing portions (6, 9) of both instrument branches (2, 3) each have a central bearing area (11) in which the two instrument branches (2, 3) overlap in a functional position and a manufacturing position, and lateral bearing areas (12, 13) adjacent on both sides, in which the two instrument branches (2, 3) only overlap when they are in an angular position other than the manufacturing position.

9. Method according to claim 8, **characterized in that** the instrument (1) is manufactured by means of laser sintering.

10. Method according to claim 8 or 9, **characterized in that** a gap of approximately 0.3 mm to 0.5 mm is produced between the first instrument branch (3) and the second instrument branch (2).

## Revendications

1. Instrument médicotechnique (1) comprenant au moins une première branche d'instrument (3) et une seconde branche d'instrument (2) qui sont mobiles l'une par rapport à l'autre, dans lequel la première branche d'instrument (3) est conçue en tant que branche d'instrument mâle (3) avec une section de palier mâle (9), et la seconde branche d'instrument (2) est conçue en tant que branche d'instrument femelle (2) avec une section de palier femelle (6) présentant une ouverture de passage (10), dans lequel la première branche d'instrument (3) est disposée sur la seconde branche d'instrument (2) de sorte que la section de palier mâle (9) traverse l'ouverture de passage (10) de la section de palier femelle (6), dans lequel l'instrument (1) est fabriqué au moyen d'un procédé de fabrication additive, et un jeu est présent entre la section de palier mâle (9) de la première branche d'instrument (3) et la section de palier femelle (6) de la seconde branche d'instrument (2) dans une position relative de la première branche d'instrument (3) par rapport à la seconde branche d'instrument (2), de sorte que la première branche d'instrument (3) et la seconde branche d'instrument (2) soient disposées sans contact l'une par rapport à l'autre,
dans lequel l'une des branches d'instrument (2, 3) présente une saillie de guidage (15) s'élevant en direction de l'autre branche d'instrument (2, 3), saillie qui s'engage dans un évidement en forme d'arc de cercle (16) conçu de manière correspondante sur l'autre branche d'instrument (2, 3) pour le positionnement pivotant des branches d'instrument (2, 3).

2. Instrument médicotechnique (1) selon la revendication 1, **caractérisé en ce que** la section de palier femelle (6) entoure complètement l'ouverture de passage (10).

3. Instrument médicotechnique (1) selon l'une quelconque des revendications 1 ou 3, **caractérisé en ce que** la zone (11) de section de palier mâle (9) recouverte par la section de palier femelle (6) dans la position de fabrication présente une hauteur (h) inférieure à l'ouverture de passage (10).

4. Instrument médicotechnique (1) selon l'une quelconque des revendications précédentes, **caractérisé en ce qu'** un angle α d'env. 80° à env. 120°, de préférence d'env. 85° à env. 115° et de préférence d'env. 90° à env. 110° est présent entre la première branche d'instrument (3) et la seconde branche d'instrument (2) dans la position de fabrication.

5. Instrument médicotechnique (1) selon l'une quelconque des revendications précédentes, **caractérisé en ce que** des structures de contact (12, 13, 17, 18) sont formées au niveau de la première branche d'instrument (3) et/ou de la seconde branche d'instrument (2) pour la mise en contact mutuelle de la première branche d'instrument (3) et de la seconde branche d'instrument (2) dans une position d'utilisation différente de la position de fabrication.

6. Instrument médicotechnique (1) selon la revendication 1, **caractérisé en ce que** l'évidement (16) est formé au niveau de la section de palier femelle (6), en particulier des deux côtés, et la saillie de guidage (15) est formée au niveau de la section de palier mâle (9), en particulier des deux côtés.

7. Instrument médicotechnique (1) selon l'une quelconque des revendications précédentes, **caractérisé en ce que** celui-ci présente une structure d'arrêt qui est conçue de sorte que, lors du transfert de l'instrument (1) d'une position d'utilisation à la position de fabrication, un arrêt du mouvement relatif des deux branches d'instrument (2, 3) est provoqué, arrêt qui doit être surmonté par une force d'actionnement accrue côté utilisateur.

8. Procédé de fabrication d'un instrument médicotechnique (1) au moyen d'un procédé de fabrication additive, dans lequel au moins une première branche d'instrument mâle (3) est formée avec une section de palier mâle (9) et une seconde branche d'instrument (2) est formée en tant que branche d'instrument femelle (2) avec une section de palier femelle (6) présentant une ouverture de passage (10), dans lequel la section de palier mâle (9) est fabriquée de manière additive en traversant l'ouverture de passage (10) de la section de palier femelle (6), un jeu est présent entre la section de palier mâle (9) de la première branche d'instrument (3) et la section de palier femelle (6) de la seconde branche d'instrument (2) dans une position relative de la première branche d'instrument (3) par rapport à la seconde branche d'instrument (2), de sorte que la première branche d'instrument (3) et la seconde branche d'instrument (2) soient disposées sans contact l'une par rapport à l'autre, et dans lequel les sections de palier (6, 9) des deux branches d'instrument (2, 3) présentent respectivement une zone de palier centrale (11) dans laquelle les deux branches d'instrument (2, 3) se chevauchent dans une position de fonctionnement et une position de fabrication et présentent des zones de palier latérales adjacentes des deux côtés (12, 13) dans lesquelles les deux branches d'instrument (2, 3) se chevauchent uniquement lorsqu'elles se trouvent dans une position angulaire différente de la position de fabrication.

9. Procédé selon la revendication 8, **caractérisé en ce que** l'instrument (1) est fabriqué par frittage sélectif au laser.

10. Procédé selon la revendication 8 ou 9, **caractérisé en ce qu'**une fente d'env. 0,3 mm à 0,5 mm est générée entre la première branche d'instrument (3) et la seconde branche d'instrument (3).
